# EUROPEAN PATENT APPLICATION

(11) **EP 4 382 132 A1**
(43) Date of publication of application: **12.06.2024**
(21) Application number: 22853567.0
(22) Date of filing: 05.08.2022
(51) Int. Cl.: A61K 48/00, A61K 31/7088, A61K 47/42, A61P 35/00, C12N 15/113

(54) **NON-NATURAL NUCLEIC ACID LIGAND, USES THEREOF, AND PHARMACEUTICAL COMPOSITION FOR PREVENTING OR TREATING CANCER COMPRISING SAME AS ACTIVE INGREDIENT**

(30) Priority: 06.08.2021 KR 20210104105
(71) Applicant: Interoligo Corporation, Gyeonggi-do 14058 (KR)
(72) Inventor: LEE, Jung Hwan, Yongin-si, Gyeonggi-do 16803 (KR); LEE, Jongook, Seongnam-si, Gyeonggi-do 13457 (KR); PARK, Hanseul, Seoul 07026 (KR); LEE, Se Na, Anyang-si, Gyeonggi-do 14016 (KR)
(74) Representative: Kilger, Ute
(86) International application number: PCT/KR2022/011718
(87) International publication number: WO 2023/014192

(57) **Abstract**

One aspect of the present disclosure relates to a novel non-natural nucleic acid ligand that binds to human serum albumin (HSA), and to uses thereof. The novel non-natural nucleic acid ligand may include the nucleic acid sequence of SEQ ID NO: 8 (CAGG6CGAGC6G6ACGCG6G6G6GACC) [wherein the moiety indicated by 6 is 5-[N-(1-naphthylmethyl)carboxamide]-2'-deoxyuridine (Nap-dU)]. Meanwhile, one aspect of the present disclosure relates to a pharmaceutical composition for treating cancer, comprising a novel non-natural nucleic acid ligand that binds to human serum albumin as an active ingredient. The pharmaceutical composition exhibits excellent effects in the treatment of cancer.

## Description

### TECHNICAL FIELD

The present invention relates to a novel non-natural nucleic acid ligand, a use thereof, and a pharmaceutical composition including same as an active ingredient for the prevention and treatment of cancer.

### BACKGROUND

According to the World Health Organization (WHO), cancer is one of the four major fatal non-communicable diseases worldwide. About ten million new cases are diagnosed each year, accounting for approximately 12% of all causes of death, which makes it the third leading cause of death.

In particular, pancreatic cancer is notably challenging to detect early because the pancreas is surrounded by many organs such as the stomach, duodenum, small intestine, liver, and gallbladder. Additionally, pancreatic cancer often does not produce noticeable symptoms, progresses rapidly, and tends to metastasize to other organs, making its prognosis significantly poorer compared to other cancers. According to cancer mortality statistics by site, pancreatic cancer has the lowest five-year relative survival rate. Although 15-20% of patients with pancreatic cancer are considered surgically curable, the majority of patients who do not undergo surgery are deemed to have locally advanced or metastatic disease. Treatment options for patients with pancreatic cancer are very limited, and there is a high problem of resistance to chemotherapy. As pancreatic cancer easily metastasizes to other organs, there is a need for early detection and treatment. Therefore, it is crucial to develop new therapeutic approaches that can target pancreatic cancer for early diagnosis and active treatment.

Additionally, breast cancer is the most common malignant tumor in women, causing over 40,000 deaths annually. One in nine women will develop breast cancer during their lifetime, and the number of breast cancer patients is increasing by about 15% each year. In Korea, as of 1995, breast cancer accounted for about 11.9% of female cancer patients, becoming the third most common cancer after cervical and stomach cancer and the fifth most deadly cancer following stomach, liver, cervical, and lung cancer, with its frequency increasing annually. Early diagnosis is crucial, but despite many known anticancer treatments, the survival rate has not significantly improved in cases of advanced, metastasized, or more dangerous types of cancer.

Particularly, triple-negative breast cancer (TNBC) accounts for 10-20% of all breast cancer cases. TNBC is defined by the lack of expression of estrogen receptors (ER) and progesterone receptors (PR) and overexpression or amplification of human epidermal growth factor receptor 2 (HER-2). It is a heterogeneous group of breast cancer tumors diagnosed through immunohistochemistry. TNBC is characterized by aggressive clinical behavior and poor prognosis due to rapid resistance to many chemotherapeutic drugs and a lack of suitable targets. TNBC patients generally have a worse prognosis compared to other breast cancer types, with higher probabilities of early recurrence in distant organs and increased mortality rates. Currently, there are no approved targeted therapies available. Classic microtubule-targeting drugs (MTDs) like paclitaxel and its semi-synthetic derivatives have achieved significant success in clinical control of breast neoplasms. Anthracycline and taxane-based chemotherapy are standard therapies for TNBC. However, most TNBC patients eventually develop drug resistance, tumor relapse, and/or metastasis after temporary responses to initial treatment. Developing drugs to effectively and significantly inhibit the primary or metastatic progression of TNBC, especially metastasis to other organs (notably the lungs), has been challenging. There is an urgent need to develop innovative and more effective therapeutic approaches that achieve more sustained responses in treating TNBC.

Other common types of cancer globally include lung, colorectal, ovarian, endometrial, cervical, bladder, small intestine, testicular, thyroid, liver, urothelial, gallbladder and biliary, skin, gastric, brain tumors, renal, and acute myeloid leukemia. New therapeutic approaches are constantly needed for the diagnosis and treatment of these cancers as well.

Meanwhile, the present inventors have discovered and optimized novel aptamers (nucleic acid ligands) through SELEX and found that the non-natrual nucleic acid ligands have excellent targeted therapeutic effects (primary and metastatic cancer treatment by intravenous/intraperitoneal administration) especially for difficult-to-treat and poor-prognosis cancers like pancreatic cancer and TNBC.

Accordingly, a new alternative to existing treatments is suggested herein, and pharmaceuticals containing these novel aptamers are expected to be effective in treating various types of cancer.

### [Related Art Documents]

### [Patent Literatures]

(Patent literature 1) Korean Patent No. 2019-0126356 A
(Patent literature 2) U.S. Patent No. 8,969,318

### [Non- Patent Literatures]

(Non-patent literature 1) Zhang et al., Recent Advances in Aptamer Discovery and Applications, Molecules 2019, 24, 941
(Non-patent literature 2) Zhou, J.; Rossi, J. Aptamers as Targeted Therapeutics: Current Potential and Challenges. Nat. Rev. Drug Discovery 2017, 16, 181-202.
(Non-patent literature 3) Dougan, H.; Lyster, D. M.; Vo, C. V.; Stafford, A.; Weitz, J. I.; Hobbs, J. B. Extending the Lifetime of Anticoagulant Oligodeoxynucleotide Aptamers in Blood. Nucl. Med. Biol. 2000, 27, 289-297.
(Non-patent literature 4) Povsic, T. J., et al. Pre-existing anti-PEG antibodies are associated with severe immediate allergic reactions to pegnivacogin, a PEGylated aptamer. J ALLERGY CLIN IMMUNOL, 2016, 138(6), 1712
(Non-patent literature 5) M. M. Soldevilla, H. Villanueva, F. Pastor, Aptamers: A Feasible Technology in Cancer Immunotherapy, Journal of Immunology Research, vol. 2016, 2016.

### SUMMARY

Leading to the present disclosure, intensive and thorough research conducted by the present inventors has discovered a novel nucleic acid ligand and confirmed its therapeutic effects in various types of cancer.

Despite extensive research for a long period of time, traditional SELEX methods and the aptamers developed therethrough have shown difficult-to-overcome limitations, making it challenging to provide aptamer-based therapeutics. In fact, there has not been a single successful development since Macugen in the past 15 years.

The present disclosure aims to provide a novel nucleic acid ligand, a use thereof, and a pharmaceutical composition containing same as an active ingredient for the treatment of cancer.

The present disclosure also aims to provide a pharmaceutical composition with excellent in vivo stability and anticancer effects.

Specifically, the present disclosure pertains to a nucleic acid ligand that binds to plasma proteins (e.g., albumin). When administered into the bloodstream, the ligand binds to plasma albumin proteins, thereby avoiding degradation by nucleases in the body or renal excretion, and can reach cancer tissues. Once reaching the cancer tissue, the ligand can enter the cells through pathways such as endocytosis and exert the intended therapeutic effects. Thus, the nucleic acid ligand according to an embodiment of the present disclosure solves the problems associated with traditional aptamers, such as in-vivo instability, rapid renal clearance, and inability to be delivered into cells.
1. An embodiment of the present disclosure pertains to a non-natural nucleic acid ligand binding to human serum albumin (HSA), which includes the nucleotide sequence of SEQ ID NO: 8 (CAGG6CGAGC6G6ACGCG6G6G6GACC) [wherein the portions marked as 6 are each 5-[N-(1-naphthylmethyl)carboxamide]-2'-deoxyuridine (Nap-dU)].
2. In an embodiment, the non-natural nucleic acid ligand may have a nucleic acid sequence selected from SEQ ID NOS: 1 to 8.
3. An embodiment of the present disclosure pertains to a pharmaceutical composition for the prevention or treatment of cancer, containing, as an active ingredient, a non-natural nucleic acid ligand or a pharmaceutically acceptable salt thereof that binds to human serum albumin (HSA), wherein the non-natural nucleic acid ligand includes the nucleic acid sequence of SEQ ID NO: 8 (CAGG6CGAGC6G6ACGCG6G6G6GACC) [wherein the portions marked as 6 are each 5-[N-(1-naphthylmethyl)carboxamide]-2'-deoxyuridine (Nap-dU)].
4. In an embodiment, the composition may include one or more of the non-natural nucleic acid ligands.
5. In an embodiment, the non-natural nucleic acid ligand containing the nucleic acid sequence of SEQ ID NO: 8 may have a nucleic acid sequence selected from SEQ ID NOS: 1 to 8.
6. In an embodiment, the cancer can be primary or metastatic.
7. In an embodiment, the cancer may be breast cancer, pancreatic cancer, lung cancer, colon cancer, ovarian cancer, endometrial cancer, cervical cancer, bladder cancer, small intestine cancer, testicular cancer, thyroid cancer, liver cancer, urothelial carcinoma, gallbladder and biliary tract cancer, skin cancer, gastric cancer, brain tumor, kidney cancer, and/or acute myeloid leukemia.
8. In an embodiment, the breast cancer may be triple-negative breast cancer.
9. In an embodiment, the cancer may be pancreatic cancer.
10. In an embodiment, the pharmaceutical composition may be administered intravenously, intramuscularly, intraarterially, intraperitoneally, intranasally, intravaginally, intravesically, intradermally, transdermally, topically, or subcutaneously to a subject in need thereof.
11. In an embodiment, the pharmaceutical composition may be administered after the surgical resection of the cancer tissue.
12. An embodiment of the present disclosure relates to a method of preventing or treating cancer, the method including a step of administering to a subject in need thereof a pharmaceutical composition containing a human serum albumin (HSA)-binding non-natural nucleic acid ligand or a pharmaceutically acceptable salt thereof according to an embodiment of the present disclosure.

According to an embodiment of the present disclosure, the novel nucleic acid ligand may be a single nucleic acid ligand that exhibits excellent specific binding affinity for a target.

The novel nucleic acid ligand according to an embodiment, when administered as a drug in the body, can specifically bind to plasma proteins, demonstrating effects such as excellent in vivo stability, slow renal clearance, and the ability to be delivered into cells through pathways such as endocytosis. With these characteristics, the novel nucleic acid ligand can exhibit excellent therapeutic efficacy for diseases dependent on therapeutic targets, finding applications as a drug in actual clinical settings.

The novel nucleic acid ligand according to an embodiment does not require use with other macromolecules (e.g., PEG) to achieve in vivo stability or slow renal clearance, thereby avoiding issues where these molecules might affect the structure of the nucleic acid ligand or reduce its affinity or specificity for the intended target. Consequently, the nucleic acid ligand can maintain its excellent therapeutic efficacy from experimental findings as a drug in clinical applications.

The novel nucleic acid ligand, according to an embodiment has a relatively short sequence length (e.g., about 20 to about 100 nucleotides, preferably about 25 to about 75 oligonucleotides) and does not require use with other substances. This ensures that the quality or performance of the nucleic acid ligand does not vary between batches in mass production processes, making quality control easier and satisfying the purity and synthesis yield required for use as a drug post-mass production. Consequently, the novel nucleic acid ligand according to an embodiment can be efficiently mass-produced using only an automatic synthesizer.

The novel nucleic acid ligand can bind to the three-dimensional structure of proteins, exhibiting excellent therapeutic effects that it aims to achieve.

The pharmaceutical composition according to an embodiment of the present disclosure includes the novel nucleic acid ligand with specific binding affinity for plasma proteins and thus can effectively and excellently prevent or treat various cancers.

The pharmaceutical composition, according to an embodiment can be administered via non-oral routes (e.g., intravenously or intraperitoneally) to effectively inhibit the growth of primary or metastatic tumors or cancers.

The pharmaceutical composition according to an embodiment can inhibit the growth of metastatic tumors or cancers post-surgical resection or excellently inhibit the metastasis of cancer or tumors.

The pharmaceutical composition according to an embodiment can be administered via non-oral routes (e.g., intravenously or intraperitoneally) to effectively and excellently prevent or treat primary or metastatic triple-negative breast cancer (TNBC) or pancreatic cancer.

### BRIEF DESCRIPTION OF DRAWINGS

FIG. 1 illustrates the results of the filter binding assay to measure the binding affinity of a nucleic acid library pool for human serum albumin (HSA) according to an embodiment in terms of an image (FIG. 1A) and a plot (FIG. 1B).
FIG. 2a illustrates the results of the filter binding assay to measure the binding affinity of nucleic acid library clones (Clones 1 to 5) for HSA according to an embodiment in terms of an image (FIG. 2A) and a plot (FIG. 2B).
FIG. 2b presents the results of the ELISA Kd assay to measure the binding affinity of nucleic acid library clones (Clones 6 to 11) for HSA according to an embodiment.
FIG. 3 illustrates the predicted secondary structures of optimized nucleic acid ligands AL001 (A in FIG. 3), AL002 (B in FIG. 3), AL003 (C in FIG. 3), AL004 (D in FIG. 3), AL005 (E in FIG. 3), and AL006 (F in FIG. 3), all being optimized from the nucleic acid ligand (AL007) that was selected as a full-length nucleic acid ligand with excellent binding affinity to HSA from the nucleic acid library pool and clones of the present disclosure.
FIG. 4 presents the results of the ELISA Kd assay to measure the binding affinity of full-length or optimized nucleic acid ligands (AL002, AL003, AL005, AL006, and AL007) of the present disclosure for HSA.
FIG. 5 illustrates the results of an in vitro anticancer efficacy of the nucleic acid ligand (AL006) of the present disclosure against the pancreatic cancer cell line (Miapaca-2).
FIG. 6 outlines the experimental scheme for the in vivo anticancer efficacy experiment with the nucleic acid ligand (AL006) of the present disclosure in a pancreatic cancer animal model
FIG. 7 depicts the results of an in vivo anticancer efficacy experiment with the nucleic acid ligand (AL006) of the present disclosure in a pancreatic cancer animal model. Specifically, FIG. 7A is a plot representing the relative tumor size change in negative or positive control drug-treated groups (PBS or gemcitabine) and AL006-treated groups, and FIG. 7B shows photographic images of the actual tumor tissues.
FIG. 8 presents in vivo toxicity due to drug administration (heart (FIG. 8A), spleen (FIG. 8B), liver (FIG. 8C), lungs (FIG. 8D), and kidneys (FIG. 8E)) after an in vivo anticancer efficacy experiment with the nucleic acid ligand (AL006) of the present disclosure in a pancreatic cancer animal model.
FIG. 9 presents in vivo toxicity due to drug administration (white blood cells (FIG. 9A), red blood cells (FIG. 9B), platelets (FIG. 9C), AST levels (FIG. 9D), and ALT levels (FIG. 9E)) after an in vivo anticancer efficacy experiment with the nucleic acid ligand (AL006) of the present disclosure in a pancreatic cancer animal model.

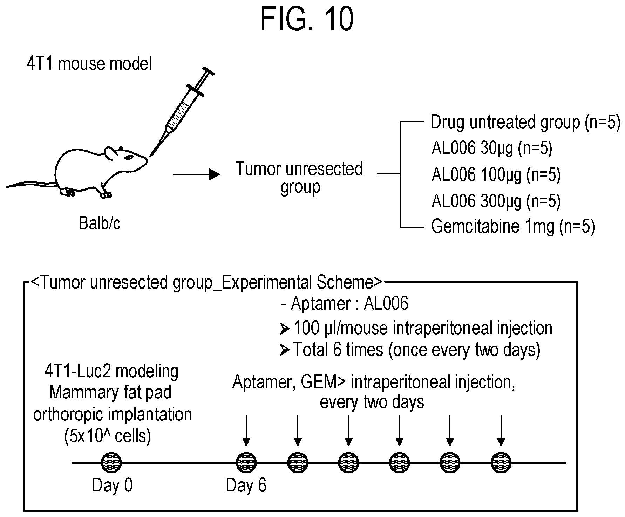

FIG. 10 is a schematic diagram illustrating an experimental overview of evaluating the anticancer efficacy of a pharmaceutical composition containing the nucleic acid ligand AL006 according to an embodiment in a triple-negative breast cancer model (TNBC mouse model).
FIG. 11 presents the results of an experiment measuring tumor size after administering varying doses of a pharmaceutical composition containing the nucleic acid ligand AL006 according to an embodiment in a triple-negative breast cancer model (TNBC mouse model) to evaluate the anticancer efficacy.
FIG. 12 shows the results of an experiment examining tumor inhibition efficacy by low-dose treatment of the human serum albumin-targeted nucleic acid ligand in an orthotopic transplantation animal model of triple-negative breast cancer.
FIG. 13 shows the results of an experiment examining tumor inhibition efficacy by high-dose treatment of the human serum albumin-targeted nucleic acid ligand in an orthotopic transplantation animal model of triple-negative breast cancer. Gemcitabine (1mg) was used as a positive control, and a Her2-targeted nucleic acid ligand (300ug) was used as a negative control.

### DETAILED DESCRIPTION

Various embodiments described herein are illustrated for the purpose of clarifying the technical idea of the present disclosure, and are not intended to limit the present disclosure to any specific embodiment. The technical idea of the present disclosure includes various modifications, equivalents, alternatives and embodiments selectively combined from all or part of individual embodiments described in the present document. Further, the scope of the technical idea of the present disclosure is not limited to the various embodiments described below, and the detailed description thereof.

The terms used herein, including technical or scientific terms, may have meanings that are generally understood by a person having ordinary knowledge in the art to which the present disclosure pertains, unless otherwise specified. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

The singular of an expression used herein may include the meaning of the plural of the expression unless otherwise indicated in the context clearly dictates otherwise, and the same applies to singular forms of expressions as set forth in the claims.

The expressions "1st", "2nd", "first", "second", and the like used herein are used to distinguish one object from another in referring to plural same objects unless otherwise indicated in the context, and do not limit the order or importance of the objects.

The expressions used herein, "A, B and C", "A, B or C", "A, B and/or C", "at least one of A, B, and C", "at least one of A, B, or C", "at least one of A, B, and/or C", "at least one selected from A, B, and C", "at least one selected from A, B, or C", "at least one selected from A, B, and/C", and the like may be used to refer to each listed item or any possible combination of the listed items may be provided. For example, the expression "at least one selected from A and B" may refer to all of (1) A, (2) at least one of A, (3) B, (4) at least one of B, (5) at least one of A and at least one of B, (6) B and at least one of A, (7) A and at least one of B, and (8) both of A and B.

The term "about" or "approximately" used herein may refer to the usual error range for each value, as is widely known to a person skilled in the art. In the context of a numerical value or range described herein, the term may mean±20%, ±15%, ±10%, ±9%, ±8%, ±7%, ±6%, ±5%, ±4%, ±3%, ±2%, or ±1% of a numerical value or range recited or claimed in an embodiment.

As used herein, the term "aptamer" refers to an oligonucleotide that has specific binding affinity for only one target.

As used herein, the terms "SELEX", "SELEX technology", "SELEX method", "SELEX process" may be used interchangeably with each other, and may refer to a combined method of a process of selecting a nucleic acid interacting with a target in a preferred manner (e.g., binding to a protein) and a process of amplifying the selected nucleic acid. This SELEX method is an in vitro method for selecting a nucleic acid molecule capable of binding to a target molecule with high specificity, which is disclosed in U.S. Pat. No. 5,475,096 ("Nucleic Acid Ligands") and U.S. Pat. No. 5,270,163 (WO 91/19813, "Nucleic Acid Ligands").

The present disclosure in an embodiment relates to a novel non-natural nucleic acid ligand, which is a new class of nucleic acid compound that was previously considered impossible to exist.

As used herein, the terms "novel nucleic acid ligand", "nucleic acid ligand", "non-natural nucleic acid ligand", and "novel non-natural nucleic acid ligand" may be used interchangeably with one another.

As used herein, the term "non-natural" refers to non-occurring in nature, and in this aspect, the novel nucleic acid ligand according to an embodiment of the present disclosure may not be a nucleic acid with a known physiological function binding to a target.

In an embodiment, the novel nucleic acid ligand may have specific binding affinity for plasma proteins (e.g., human serum albumin).

As used herein, the term "specific binding affinity" may mean that the nucleic acid ligand binds to its target with generally much higher affinity than binding to other non-target components or ingredients present in a mixture or sample.

In an embodiment, the targets may have a three-dimensional structure. In an embodiment, the targets do not include a polynucleotide that binds to a nucleic acid through a mechanism which predominantly depends on Watson/Crick base pairing or triple helix binding.

In an embodiment, the novel nucleic acid ligand may interact with the targets by three-dimensionally binding to the targets, recognizing three-dimensional structures of the targets, or forming three-dimensional structures by itself. The novel nucleic acid ligand may regulate characteristics of respective targets by binding to the targets, recognizing three-dimensional structures of the targets, or interacting with the targets. For example, a novel nucleic acid ligand according to an example can bind to a target protein to inhibit the activity of a protein, block signaling pathways, or inhibit activity of proteins downstream of signaling pathways.

As used herein, the term "binding" or "interacting" may refer to the association between two molecules (e.g., a stable association between a nucleic acid ligand and a target) due to, for example, pi-stacking, electrostatic interactions, hydrophobic interactions, ionic interactions, and/or hydrogen bonding interactions, under physiological conditions.

As used herein, the term "regulating" may refer to changing functional characteristics, biological activity, and/or biological mechanisms (or pathways) of targets. For example, the term may refer to upregulating (e.g., activating or stimulating), downregulating (e.g., inhibiting or suppressing), or changing characteristics, activity, and/or mechanisms of targets. By way of example, when the target is a cell, the characteristics of the cell to be regulated may be cell viability, cell proliferation, gene expression, cell morphology, cell activation, phosphorylation, calcium mobilization, degranulation, cell migration, and/or cell differentiation.

In an embodiment, the novel nucleic acid ligand may consist of about 100 or less nucleotides. Specifically, the novel nucleic acid ligand may consist of 100 or less, 90 or less, 80 or less, 70 or less, 60 or less, 50 or less, 40 or less, or 30 or less nucleotides. In an embodiment, the novel nucleic acid ligand may consist of 10 to 105, 10 to 100, 15 to 95, 15 to 90, 20 to 85, 20 to 80, 25 to 75, 25 to 70, 25 to 65, or 25 to 60 nucleotides.

In an embodiment, the novel nucleic acid ligand may include the nucleic acid sequence of SEQ ID NO: 8 (CAGG6CGAGC6G6ACGCG6G6G6GACC) [wherein, the portions marked as "6" may each be 5-[N-(1-naphthylmethyl)carboxamide]-2'-deoxyuridine(Nap-dU)]. Specifically, the novel nucleic acid ligand may include any one nucleic acid sequence selected from the group consisting of SEQ ID NOS: 1 to 8.

In an embodiment, the nucleotides may be selected from the group consisting of DNA nucleotides, RNA nucleotides, modified nucleotides thereof, and combinations thereof.

The modified nucleotide may be linked to the 5' end, 3' end or any one or more nucleotides located in the oligonucleotide, with preference for the 5' end of the oligonucleotide.

As used herein, the term "modified nucleotide" may refer to an analog, substituent, or ester of a naturally occurring nucleotide (A, G, T/U, and C), and may mean a broad concept encompassing modified nucleotides easily available by a person skilled in the art. For example, the modified nucleotide may refer to a nucleotide having a substitution at the C-5 position (e.g., a C-5 modified pyrimidine), a nucleotide having a modified sugar (ribose or deoxyribose) constituting the nucleotide, or a nucleotide having all of such modifications.

In an embodiment, the C-5 modified pyrimidine may refer to a C-5 modified amino carbonyl pyrimidine, and examples thereof may include 5-(N-benzylcarboxyamide)-2'-deoxyuridine (Bz-dU), 5-(N-isobutylcarboxyamide)-2'-deoxyuridine (iBu-dU), 5-(N-phenethylcarboxyamide)-2'-deoxyuridine (Pe-dU), 5-(N-thiophenylmethylcarboxyamide)-2'-deoxyuridine (Th-dU), 5-(N-[2-(1H-indol-3yl)ethyl]carboxyamide)-2'-deoxyuridine (Trp-dU), 5-(N-[1-(3-trimethylammonium)propyl]carboxyamide)-2'-deoxyuridine chloride, 5-(N-naphthylmethylcarboxyamide)-2'-deoxyuridine (Nap-dU), 5-(N-[1-(2,3-dihydroxypropyl)]carboxyamide)-2'-deoxyuridine), 5-(N-benzylcarboxyamide)-2'-deoxycytidine (Bz-dC), 5-(N-isobutylcarboxyamide)-2'-deoxycytidine (iBu-dC), 5-(N-phenethylcarboxyamide)-2'-deoxycytidine (Pe-dC), 5-(N-thiophenylmethylcarboxyamide)-2'-deoxycytidine (Th-dC), 5-(N-[2-(1H-indol-3yl)ethyl]carboxyamide)-2'-deoxycytidine (Trp-dC), 5-(N-[1-(3-trimethylammonium)propyl]carboxyamide)-2'-deoxycytidine chloride, 5-(N-naphthylmethylcarboxyamide)-2'-deoxycytidine (Nap-dC), and/or 5-(N-[1-(2,3-dihydroxypropyl)]carboxyamide)-2'-deoxycytidine, but is not limited thereto.

In an embodiment, a modified nucleotide having a 2'-position modification of the ribose sugar may include 2'-deoxy-2'-fluorouridine (Uf), 2'-deoxy-2'-fluorocytidine (Cf), 2'-hydroxyadenosine (Ar), 2'-methoxyadenosine (Am), and 2'-methoxyguanosine (Gm), but with no limitations thereto.

In an embodiment, a modified nucleotide having both a C-5 modification and a *2'* position modification of the ribose sugar may include 5-(N-benzylcarboxyamide)-2'-O-methyluridine, 5-(N-benzylcarboxyamide)-2'-fluorouridine, 5-(N-isobutylcarboxyamide)-2'-O-methyluridine, 5-(N-isobutylcarboxyamide)-2'-fluorouridine, 5-(N-[2-(1H-indol-3yl)ethyl]carboxyamide)-2'-O-methyluridine, 5-(N-[2-(1H-indol-3yl)ethyl]carboxyamide)-2'-O-fluorouridine, 5-(N-naphthylmethylcarboxyamide)-2'-O-methyluridine, 5-(N-naphthylmethylcarboxyamide)-2'-fluorouridine, 5-(N-[1-(2,3-dihydroxypropyl)]carboxyamide)-2'-O-methyluridine, and/or 5-(N-[1-(2,3-dihydroxypropyl)]carboxyamide)-2'-O-fluorouridine, but with no limitations thereto.

In an embodiment, the modified nucleotide having both a C-5 modification and a 2*'* position modification of the ribose sugar may include 5-(N-benzylcarboxyamide)-2'-O-methylcytidine, 5-(N-benzylcarboxyamide)-2'-fluorocytidine, 5-(N-isobutylcarboxyamide)-2'-O-methylcytidine, 5-(N-isobutylcarboxyamide)-2'-fluorocytidine, 5-(N-[2-(1H-indol-3yl)ethyl]carboxyamide)-2'-O-methylcytidine, 5-(N-[2-(1H-indol-3yl)ethyl]carboxyamide)-2'-O-fluorocytidine, 5-(N-naphthylmethylcarboxyamide)-2'-O-methylcytidine, 5-(N-naphthylmethylcarboxyamide)-2'-fluorocytidine, 5-(N-[1-(2,3-dihydroxypropyl)]carboxyamide)-2'-O-methylcytidine, and/or 5-(N-[1-(2,3-dihydroxypropyl)]carboxyamide)-2'-O-fluorocytidine, but is not limited thereto.

In an embodiment, the modified nucleotide may be 5-(N-benzylcarboxyamide)-2'-deoxyuridine (Bz-dU) or 5-(N-naphthylmethylcarboxyamide)-2'-deoxyuridine (Nap-dU). Thus, at least one of the nucleotides constituting the novel nucleic acid ligand may be such a modified nucleotide.

In an embodiment, the novel nucleic acid ligand may consist of one nucleic acid sequence selected from the group consisting of SEQ ID NO: 1 to SEQ ID NO: 8.

In an embodiment, the novel nucleic acid ligand may be selected from the group consisting of single-stranded RNA, double-stranded RNA, single-stranded DNA, and double-stranded DNA.

In an embodiment, the novel nucleic acid ligand may be used by conjugating biotin in front of the 5'-end or the back of the 3'-end as needed.

In an embodiment, the novel nucleic acid ligand may be used by conjugating a fluorescent substance (e.g., FAM, VIC, HEX, BHQ-1, Cy5, Cy3, rhodamine, Texas Red, etc.) in front of the 5'-end or the back of the 3'-end as needed.

When modified nucleotides are attached to the 3' end of an oligonucleotide, and additional elements such as inverted dT (idT), locked nucleic acid (LNA), polyethylene glycol (PEG), or 2'-methoxy nucleosides (2'OMeNu) may be linked to the 3' end of the modified nucleotides.

The additional element idT, LNA, PEG, or 2'OMeNu linked to the 3' end of the modified nucleotide protects the 3' end of the oligonucleotide from nucleases and reduces the degradation of the oligonucleotide in the body, allowing the modified nucleotide to remain attached to the oligonucleotide for a longer duration, potentially enhancing the anticancer efficacy thereof.

To connect oligonucleotides and modified nucleotides, one method may include a step of attaching the modified nucleotide to at least one of the 5' and 3' ends of the aforementioned oligonucleotide.

Each nucleotide in the oligonucleotide sequence of the present disclosure can be substituted with a modified nucleotide, and the oligonucleotide sequence may contain one or more modified nucleotides therein.

The oligonucleotide of the present disclosure could be a nucleic acid ligand with specific binding affinity for plasma proteins. Additionally, the nucleic acid ligand of the present disclosure may have anticancer effects. The nucleic acid ligands are shown in Table 1. In Table 1, the portion marked as "6" represents 5-[N-(1-naphthylmethyl)carboxamide]-2'-deoxyuridine (Nap-dU). Furthermore, the nucleic acid ligands below are composed of DNA, which consists of deoxyribose-based nucleic acids, where G stands for 2'-deoxyguanosine, C for 2'-deoxycytidine, A for 2'-deoxyadenosine, and T for thymidine.

**TABLE 1**

| Nucleic acid ligand code | Base sequence |
|---|---|
| AL007 | GAGTGACCGTCTGCCTGC6C6CCCAGG6CGAGC6G6ACGCG6 G6G6GACC6GACG6GCAGCCACACCACCAGCC(SEQ ID NO:: 1) |
| AL001 | GCCTGC6C6CCCAGG6CGAGC6G6ACGCG6G6G6GACC6GAC G6GCAGCC(SEQ ID NO:: 2) |
| AL002 | C6C6CCCAGG6CGAGC6G6ACGCG6G6G6GACC6GACG6G(SE Q ID NO:: 3) |
| AL003 | C6C6CCCAGG6CGAGC6G6ACGCG6G6G6GACC6G(SEQ ID NO:: 4) |
| AL004 | CCAGG6CGAGC6G6ACGCG6G6G6GACC6GACG6G(SEQ ID NO:: 5) |
| AL005 | 6CCCAGG6CGAGC6G6ACGCG6G6G6GACC(SEQ ID NO:: 6) |
| AL006 | CAGG6CGAGC6G6ACGCG6G6G6GACC6G(SEQ ID NO:: 7) |
| Common sequence | CAGG6CGAGC6G6ACGCG6G6G6GACC(SEQ ID NO:: 8) |

Additionally, the present disclosure provides a pharmaceutical composition for treating cancer, which includes the aforementioned oligonucleotide, nucleic acid ligand, or a pharmaceutically acceptable salt thereof. The oligonucleotide or nucleic acid ligand is as described in the foregoing. In an embodiment, the pharmaceutical composition can be a drug delivery composition.

In a pharmaceutical composition according to an example of the present disclosure, a novel nucleic acid may be delivered into cancer cells having a therapeutic target via a pathway of endocytosis or the like. The novel nucleic acid ligand may bind to a plasma protein present in the body of a subject to which the pharmaceutical composition is administered. Therefore, the pharmaceutical composition containing the novel nucleic acid ligand can exhibit excellent in vivo stability and slow renal clearance (excellent residence time in the body), and the novel nucleic acid ligand can enter cancer cells via a pathway of endocytosis or the like after reaching the cancer tissue.

As used herein, the term "subject" refers to mammals including humans, such as primates, mice, rats, hamsters, rabbits, horses, sheep, pigs, goats, cattle, dogs, or cats, that have or may develop a disease or a disorder caused by a target or its action, with non-limitative preference for humans.

As used herein, the term "endocytosis" refers to a cellular action by which a cell transports a substance outside the cell membrane into the cell membrane. By endocytosis, a foreign substance is sent into the cell while enclosed in the cell membrane, and the absorbed substance is degraded by vesicle formation in the cell and then recycled or discharged to the outside. Endocytosis may be divided into macropinocytosis, receptor-mediated endocytosis involving clathrin protein, caveolae endocytosis, and the like, depending on the method of activation or the type of protein involved.

In an embodiment, the pharmaceutical composition may further contain a plasma protein. In this regard, the pharmaceutical composition may be in the form of a conjugate in which the plasma protein is bound to the novel nucleic acid ligand or in the form of the novel nucleic acid ligand alone, and the plasma protein may be a protein that is present outside the body of a subject to be treated.

In a pharmaceutical composition according to an example of the present disclosure, a novel nucleic acid ligand may be delivered into cancer cells having a therapeutic target via a pathway of macropinocytosis or the like.

As used herein, the term "macropinocytosis" is one type of endocytosis, wherein a pocket may be formed while the cellular membrane protrudes out of a cell and then contracts again by the cytoskeletal protein actin and the formed pocket may be separated while entering the cell, thereby forming vesicles (macropinosomes) with a diameter of about 0.2 µm to 5 µm.

Macropinocytosis plays an important role in tumor metabolism, which recently attracts attention. Through macropinocytosis, nutrients (e.g., glutamine, albumin, etc.) necessary for rapid growth of tumor cells may be supplied into cancer cells and used for metabolism. That is, extracellular fluid, extracellular molecules, and the like may be contained inside the vesicles formed by macropinocytosis.

In an embodiment, the efficiency of delivery of the nucleic acid ligand into the cell by macropinocytosis can be increased by binding of the nucleic acid ligand and the plasma protein. That is, the nucleic acid ligand binding to the plasma protein may be excellent in the delivery into cells compared with nucleic acid ligands not binding to the plasma protein.

A nucleic acid ligand according to an embodiment in the present disclosure can be delivered into the cytoplasm through various mechanisms of endocytosis. Macropinocytosis, which has attracted attention as a main pathway for supplying essential nutrients necessary for rapid proliferation of cancer cells, as well as other endocytosis pathways may be involved in the delivery of the nucleic acid ligand.

As used herein, the term "pharmaceutically effective amount" refers to an amount sufficient to treat a disease at a reasonable benefit/risk ratio applicable to medical treatment, and the level of the effective dose may be determined depending on factors including the patient's body weight, sex, age, health condition, severity, or indication, activity of a drug, sensitivity to a drug, time of administration, route of administration, excretion rate, duration of treatment, and drugs that are simultaneously used, and other factors well known in the medical field.

The present disclosure provides a drug delivery device including the pharmaceutical composition.

Provided according to an aspect is a method for treating and/or preventing cancer by administering and/or surgically implanting the pharmaceutical composition, drug delivery composition, or drug delivery device. Another aspect provides a method of preventing primary tumor regrowth by administering and/or surgically implanting the pharmaceutical composition, drug delivery composition, or drug delivery device. A further aspect provides a method of preventing tumor recurrence and/or metastasis by administering and/or surgically implanting the pharmaceutical composition, drug delivery composition, or drug delivery device. In certain embodiments, the method further includes administering and/or surgically implanting the pharmaceutical composition, drug delivery composition, or drug delivery device after surgical resection of a cancer or tumor. In certain embodiments, the method further includes administering and/or surgically implanting the pharmaceutical composition, drug delivery composition, or drug delivery device at the site of cancer or tumor resection.

The amount of the oligonucleotide, the nucleic acid ligand, or a pharmaceutically acceptable salt thereof included in the pharmaceutical composition, drug delivery composition, or drug delivery device for cancer treatment should be effective for preventing or treating cancer. The amount can be appropriately adjusted based on the condition of the subject and/or the severity of the disease.

As used herein, the term "pharmaceutically acceptable" may mean that a certain substance is non-toxic and does not interact with the action of the active ingredient of the pharmaceutical composition, and for example, may mean those approved by the management agency of each country or listed in the pharmacopoeia of each country. As used herein, the term "pharmaceutically acceptable salt" may refer to a product that contains an ionic bond and is conventionally produced by reacting a compound with an acid or base suitable for administration of a compound to a subject.

In an exemplary embodiment, the pharmaceutical composition may be administered as an individual therapeutic agent or in combination with other therapeutic agents. For combination therapy, the pharmaceutical composition and other therapeutic agents may be administered sequentially or simultaneously, and may be administered in single or multiple doses. It is important to administer the pharmaceutical composition of the present disclosure in an amount capable of ensuring maximum effects in a minimal amount without side effects, which can be easily determined by a person skilled in the art.

Further, the present disclosure provides a method for manufacturing a pharmaceutical composition for treating the aforementioned cancers.

The method for manufacturing a pharmaceutical composition of the present disclosure includes a step of preparing a dispersion containing a nucleic acid ligand.

In an embodiment, the dispersion containing the nucleic acid ligand can be prepared by mixing the nucleic acid ligand with a pharmaceutically acceptable carrier, diluent, and/or excipient. The "pharmaceutically acceptable carrier, diluent, and/or excipient" of the present disclosure refers to any adjuvant, carrier, adjuvant, lubricant, sweetener, diluent, preservative, dye/colorant, flavor enhancer, surfactant, wetting agent, dispersant, suspending agent, stabilizer, osmotic agent, solvent, surfactant, or emulsifier approved for human use by the FDA, without limitation. Examples of pharmaceutically acceptable carriers include: sugars such as lactose, glucose, and sucrose; starches such as corn starch and potato starch; cellulose and derivatives thereof, such as sodium carboxymethylcellulose, ethyl cellulose, and cellulose acetate; tragacanth; malt; gelatin; talc; cocoa butter, waxes, animal and vegetable fats, paraffin, silicone, bentonite, silicic acid, zinc oxide; oils such as peanut oil, cottonseed oil, safflower oil, sesame oil, olive oil, corn oil, and soybean oil; glycols such as propylene glycol; polyols like glycerin, sorbitol, mannitol, and polyethylene glycol; esters such as ethyl oleate and ethyl laurate; agar; buffering agents such as magnesium hydroxide and aluminum hydroxide; alginic acid; water; isotonic saline; Ringer's solution; ethyl alcohol; phosphate buffers (e.g., PBS); and any other chemical compounds used in pharmaceutical formulations, but are not limited there.

In an embodiment, the present disclosure relates to a method of preventing or treating cancer, the method including a step of administering the novel nucleic acid ligand according to the embodiment in a pharmaceutically effective amount to a subject in need thereof.

In an embodiment, the present disclosure relates to a use of the novel nucleic acid ligand according to an embodiment for manufacturing a medicament for preventing or treating cancer.

In an embodiment, the present disclosure relates to a novel nucleic acid ligand according to an embodiment for use in preventing or treating cancer.

The pharmaceutical composition, use, and treatment methods described herein can be applied interchangeably as long as they are not contradictory.

The present disclosure will be clearly understood from the above-described embodiments and the below-described examples. Hereinafter, the present disclosure will be explained in detail such that a person skilled in the art can easily understand and implement the disclosure by way of the examples described in reference to accompanying tables. However, these examples are given for illustrating the present disclosure, and the scope of the present disclosure is not limited to these examples.

The following working examples were carried out to obtain nucleic acid ligands according to an embodiment of the present disclosure.

### [EXAMPLE 1] Selection of Novel Nucleic Acid Ligand

A novel nucleic acid ligand that binds to human serum albumin (HSA; Abcam, England, trade name: Recombinant human serum albumin Protein (His tag), catalog no.: ab217817) was identified using SELEX technology. Below is the detailed method for selecting the novel nucleic acid ligand:

### 1. Construction of Library for SELEX

The template for the nucleic acid library consisted of a 5' end primer binding site with biotin, a 3' end primer binding site, and a central region of 40 consecutive random sequences [Biotin- GCTGGTGGTGTGGCTG-N(40)-CAGGCAGACGGTCACTCA] (SEQ ID NO: 9). This nucleic acid library template was synthesized using a DNA synthesizer (TriLink, USA).

To construct a nucleic acid library containing the modified nucleotide 5-[N-(1-naphthylmethyl)carboxamide]-2'-deoxyuridine (Nap-dU), the following procedure was performed. The library in a 5M NaCl solution was reacted with 500 µl of streptavidin-modified resin (Thermo Scientific, USA) for one hour to immobilize the biotin-labeled library to the resin. To produce double-stranded DNA bearing the modified nucleotide, the mixture was reacted at 70°C for 2 hours with a 5' primer, dNTPs (dATP, dGTP, dCTP, Nap-dUTP), KOD XL DNA polymerase, Tris HCl (pH 7.8), and 1X KOD buffer. Then, the addition of 20 mM NaOH ensure the separation of the resulting double-stranded DNA into single strands, and the supernatant was neutralized with neutralization buffer (700mM HCl, 180mM HEPES, 0.45% Tween20). The neutralized library was concentrated using Amicon ultra-15 (Merck Millipore, USA) and quantified using a UV spectrophotometer.

### 2. Selection of Human Serum Albumin-Specific Nucleic Acid Library Pool

For SELEX, recombinant human serum albumin (HSA) with a histidine tag (6x His-tag) was used. Dynabeads (Thermo Scientific, USA), which are magnetic beads to which the His-tag of the protein binds, were reacted at 25°C and 1200rpm for 30 minutes with HSA recombinant proteins dissolved in buffer SB 18 (40mM HEPES, 102mM NaCl, 5mM KCl, 5mM MgCl2, 0.05% Tween 20; same hereafter), followed by three rounds of wash with buffer SB18 using a magnet to prepare bead-bound HSA.

To induce the formation of the 3D structure of the library pool, the reaction was conducted with temperature reduction from 95°C to 37°C. To prevent non-specific binding, a protein competition buffer (1uM prothrombin, 1uM casein) was mixed with the above reaction mixture before reaction with magnetic beads having HAS immobilized thereto in a thermomixer at 37°C and 1200rpm for one hour. After removing the supernatant using a magnet, the beads were washed five times with buffer SB18, and the nucleic acid library pool bound to the target protein was eluted using a 2mM NaOH solution and neutralized with an 8mM HCl solution.

The neutralized nucleic acid library was mixed with qPCR buffer [5' primer (GAGTGACCGTCTGCCTG) (SEQ ID NO: 10), 3' primer (Biotin-GCTGGTGGTGTGGCTG) (SEQ ID NO: 11), 1X KOD buffer, KOD XL DNA polymerase, dNTP] and subjected to one cycle of 96°C for 15 seconds, 55°C for 10 seconds, and 68°C for 30 minutes and then to 20 cycles of 96°C for 15 seconds and 72°C for 1 minute to produce double-stranded oligo DNA.

The double-stranded oligo DNA was fixed to streptavidin magnetic beads (Thermo Scientific, USA; same hereafter) and reacted at room temperature. The anti-sense strand was removed by adding a 20mM NaOH solution. The sense strand fixed on the magnetic beads was used as a template to synthesize the library pool using the same method as for the library construction, and the library was used in the next round of SELEX. A total of 8 rounds of SELEX were performed.

### 3. Measurement of Binding Affinity of Nucleic Acid Library Pool for Human Serum Albumin (Filter Binding Assay)

A filter binding assay was performed to measure the binding affinity of the nucleic acid library pool for human serum albumin (HSA).

First, double-stranded oligo DNA was prepared from the 6th and 8th round nucleic acid library pools by mixing with qPCR buffer [5' primer (GAGTGACCGTCTGCCTG) (SEQ ID NO: 10), 3' primer (Biotin-GCTGGTGGTGTGGCTG) (SEQ ID NO: 11), 1X KOD buffer, KOD XL DNA polymerase, dNTP], followed by 20 cycles of 96°C for 15 seconds and 72°C for 1 minute. The double-stranded oligo DNA was reacted at room temperature with and fixed to streptavidin magnetic beads. The anti-sense strand was removed with a 20mM NaOH solution. The magnetic beads deprived of the anti-sense strand were washed twice with buffer SB 17 (40 mM HEPES, 102 mM NaCl, 5 mM KCl, 5 mM MgCl2, 1 mM EDTA, and 0.05 %(v/v) Tween 20; same hereafter) and once with 16mM NaCl. The washed magnetic beads were added and reacted with the 5' primer, dNTP (dATP, dGTP, dCTP, Nap-dUTP), KOD XL DNA polymerase, Tris HCL (pH 7.8), and 1X KOD buffer at 68°C for one hour to prepare a modified nucleic acid-bearing nucleic acid library pool. Finally, the anti-sense strands were recovered by adding 20mM NaOH and neutralized with 80mM HCl.

The neutralized nucleic acid library pool (1 pmole) was mixed and then reacted with α-32P ATP, TdT, and NEBufferTM 4 (New England Biolabs, USA) at 37°C for 30 minutes to label the terminus of the nucleic acid library pool with α-32P ATP. The TdT enzyme was inactivated by treatment at 70°C for 10 minutes. The labeled nucleic acid library pool was purified using a Micro spin G50 column (GE Healthcare, USA) and quantified using a beta-counter.

Based on the quantification, 20000 cpm of the nucleic acid library pool was induced to form a 3D structure by reacting at every 1°C from 95°C to 37°C for 10 seconds each. The structurally stabilized nucleic acid library pool was reacted with human serum albumin diluted stepwise in buffer SB18 at 37°C for 30 minutes and further reacted with Zorbax resin (Agilent, USA) for an additional 5 minutes. After completion of the reaction, the sample was transferred to a MultiScreen-HV Filter plate (Merck Millipore, USA), and the solution was removed by vacuum. The samples that did not pass through the filter were washed with buffer SB18. After removing the buffer SB 18 by vacuum, the filter plate was exposed to an imaging plate for 16 hours and quantified using an FLA-5100 (Fujifilm, Japan). The values obtained from the filter binding assay were analyzed using SigmaPlot 11 analysis software, and a Nap library, which is a nucleic acid library pool that did not undergo SELEX, was used as a negative control.

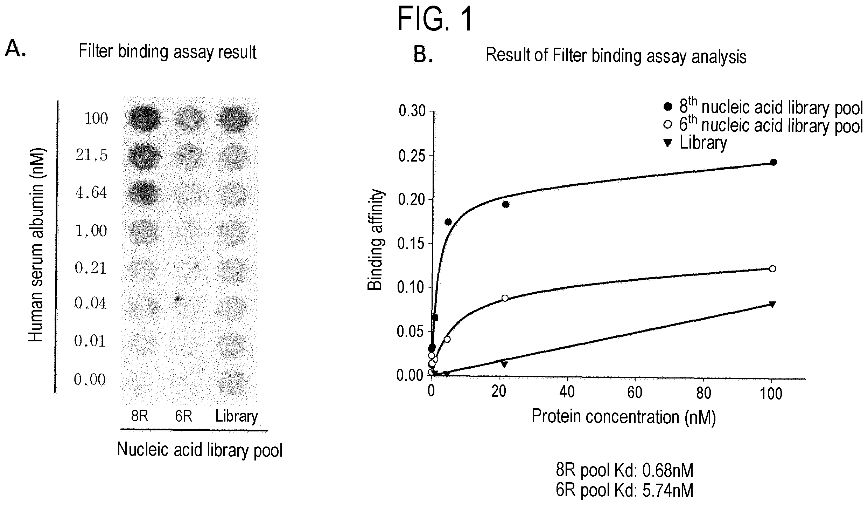

FIG. 1 shows the binding affinity of the nucleic acid library pool for human serum albumin as measured by filter binding assay. According to FIG. 1, both the 6th and 8th round nucleic acid library pools showed binding affinity for human serum protein, whereas no binding affinity was detected in the negative control Nap library.

### 4. Nucleotide Sequence Analysis of Nucleic Acid Library Pool

The nucleic acid library pool samples, which demonstrated binding affinity as measured by the filter binding assay, were amplified by PCR using the 5' primer (GAGTGACCGTCTGCCTG) (SEQ ID NO: 10) and 3' primer (GCTGGTGGTGTGGCTG) (SEQ ID NO: 12). The PCR product was cloned using the TOPO TA cloning kit and transformed into E. coli TOP10 cells. Plasmids were extracted from individual E. coli colonies, and amplified using a rolling circle amplification (RCA) kit (Enzynomics, Korea). The amplified RCA samples underwent base sequencing analysis with the aid of the BigDye terminator cycle sequencing kit (Thermo Scientific, USA) and capillary electrophoresis (Solgent, Korea). After completion of the base sequencing, the nucleic acid sequences were analyzed using in-house software to select sequences with a high frequency of expression.

The full-length nucleic acid ligands selected from the base sequencing analysis results are Clone 1, Clone 2, Clone 3, Clone 4, and Clone 5, as listed in Table 2. In the nucleotide sequences listed in Table 2, the portion indicated by "6" represents 5-[N-(1-naphthylmethyl)carboxamide]-2'-deoxyuridine (Nap-dU).

**TABLE 2**

| Nucleic acid ligand code | Sequence |
|---|---|
| Clone 1 | GAGTGACCGTCTGCCTGGAC6A6G66CGA6C6A6CACGG6C6CG6GA6 C6CGA6AGACAGCCACACCACCAGCC (SEQ ID NO: 13) |
| Clone 3 | GAGTGACCGTCTGCCTGAGGCC6GA6C66GAAGG6A6AGAAC66AA CGCGG6CG66ACAGCCACACCACCAGCC (SEQ ID NO: 14) |
| Clone 4 | GAGTGACCGTCTGCCTGAA6GGC66C6GCC666G6AACACGGCAAC6 G6CCACGGACCAGCCACACCACCAGCC (SEQ ID NO: 15) |
| Clone 5 | GAGTGACCGTCTGCCTGCCGC66A6GAG6C6CG6GAGA6GGACCG6G CGCCAGCCCCCAGCCACACCACCAGCC (SEQ ID NO: 16) |
| Clone 6 | GAGTGACCGTCTGCCT6AG6G6GA6GA6C66GACA6AGAAC66AACC GGA6GG6CACAGCCACACCACCAGCC (SEQ ID NO: 17) |
| Clone 7 | GAGTGACCGTCTGCCTGC6GAA6G6C6G6CAAA6AC6A6ACGG6C6C G6A6CA6GACAGCCACACCACCAGCC (SEQ ID NO: 18) |
| Clone 8 | GAGTGACCGTCTGCCT6AG6GGA6GA6C66GCCA6AGAAC66AACC GGA6GG6CACAGCCACACCACCAGCC (SEQ ID NO: 19) |
| Clone 9 | GAGTGACCGTCTGCCTAC6AAA6G6C6G6CAAA6AC6A6ACGG6C6C G6A6CA6GACAGCCACACCACCAGCC (SEQ ID NO: 20) |
| Clone 10 | GAGTGACCGTCTGCCT6C66GAAC6AGAAC66AACCGC6A6GG66AC AGCGG6ACAGCCACACCACCAGCC (SEQ ID NO: 21) |
| Clone 11 | GAGTGACCGTCTGCCT6AG6GGA6GA6C66GCAA6AGAAC666ACC GGA6GG66ACAGCCACACCACCAGCC (SEQ ID NO: 22) |
| Clone 2 (AL007) | GAGTGACCGTCTGCCTGC6C6CCCAGG6CGAGC6G6ACGCG6G6G6G ACC6GACG6GCAGCCACACCACCAGCC (SEQ ID NO: 1) |
| AL001 | GCCTGC6C6CCCAGG6CGAGC6G6ACGCG6G6G6GACC6GACG6GCA GCC (SEQ ID NO: 2) |
| AL002 | C6C6CCCAGG6CGAGC6G6ACGCG6G6G6GACC6GACG6G (SEQ ID NO: 3) |
| AL003 | C6C6CCCAGG6CGAGC6G6ACGCG6G6G6GACC6G (SEQ ID NO: 4) |
| AL004 | CCAGG6CGAGC6G6ACGCG6G6G6GACC6GACG6G (SEQ ID NO: 5) |
| AL005 | 6CCCAGG6CGAGC6G6ACGCG6G6G6GACC (SEQ ID NO: 6) |
| AL006 | CAGG6CGAGC6G6ACGCG6G6G6GACC6G (SEQ ID NO: 7) |

| | |
|---|---|
| *The full-length ligands are indicated in bold and underlined. **The filter binding assay results for full-length ligand Clones 1, 2, 3, 4, and 5 are shown in FIG. 2a. ***Among the full-length ligands, the optimized (truncated) sequences of Clone 2 (AL007) are AL001, AL002, AL003, AL004, AL005, and AL006. | |

### [EXAMPLE 2] Synthesis and Binding Affinity Assay of Cloned Nucleic Acid Ligands

### 2-1. Filter Binding Assay

For the nucleic acid sequences selected through the previously described nucleotide sequence analysis, clone nucleic acid ligands were synthesized by PCR in the presence of the 5' primer (GAGTGACCGTCTGCCTG) (SEQ ID NO: 10) and 3' primer (GCTGGTGGTGTGGCTG) (SEQ ID NO: 12) with the double-stranded DNA used in base sequencing analysis serving as a template. The binding affinity of cloned nucleic acid ligands for the target protein was measured using the same method as the previously described filter binding assay.

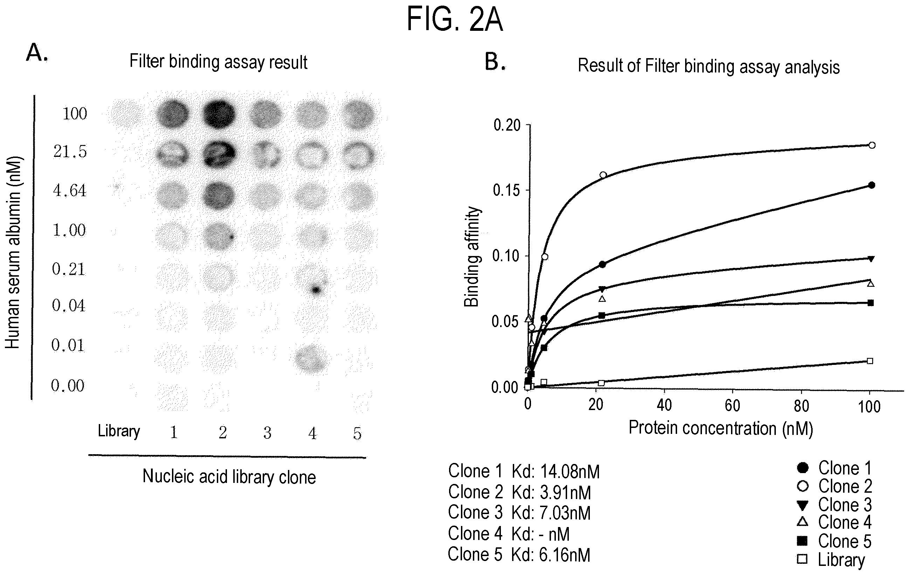

FIG. 2a shows the binding affinity of nucleic acid library clones for human serum albumin as measured by the filter binding assay. According to FIG. 2a, binding affinity for human serum albumin was confirmed for clones 1, 2, 3, and 5, while the negative control, the Nap library, showed no binding affinity.

Among the four clones with confirmed binding affinity, Clone 2, which showed the highest binding affinity, was selected as the original full-length nucleic acid ligand which was then optimized through truncation, as described below.

### 2-2. Bead-based ELISA Binding Assay

For the nucleic acid sequences selected through the previously described nucleotide sequence analysis, clone nucleic acid ligands were synthesized by PCR in the presence of 5' primer (GAGTGACCGTCTGCCTG) (SEQ ID NO: 10) and 3' primer (GCTGGTGGTGTGGCTG) (SEQ ID NO: 12), with the double-stranded DNA template used for the base sequencing analysis serving as a template.

To induce the formation of the tertiary structure of biotin-labeled nucleic acid ligands synthesized through the PCR process, the reaction was conducted while lowering the temperature gradually from 95°C to 37°C by 1°C every 10 seconds. The prepared nucleic acid ligands were allowed at 37°C for 30 minutes to bind to the human serum albumin that was 5-fold serially diluted starting from 200nM, and then histidine pull-down beads (Thermo Scientific, USA) were added and reacted for an additional 30 minutes at 25°C. The supernatant was removed using a magnet, the beads were washed three times with buffer SB 18, and then streptavidin-horseradish peroxidase (HRP) reagent diluted 1: 10,000 was added and reacted for 30 minutes at 25°C. After the supernatant was again removed using a magnet, the beads were washed three times with buffer SB 18, and then reacted with the chemiluminescent solution from the ECL Kit (Elpis, Korea). The supernatant was separated using a magnet, transferred to a white plate, and measured for fluorescence using a Glomax plate reader (Promega, USA) according to the luminescence protocol. The measurements were analyzed using SigmaPlot 11 analysis software.

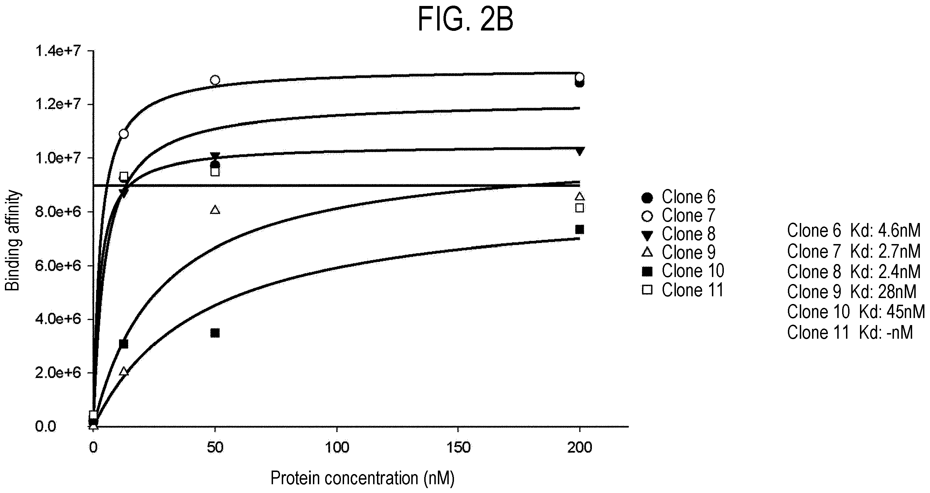

FIG. 2b shows the binding affinity of nucleic acid library clones for human serum albumin as measured by a bead-based ELISA binding assay. According to FIG. 2b, binding affinity for human serum albumin was confirmed for clones 6, 7, 8, 9, and 10.

### [EXAMPLE 3] Optimization (Truncation) of Full-Length Nucleic Acid Ligand

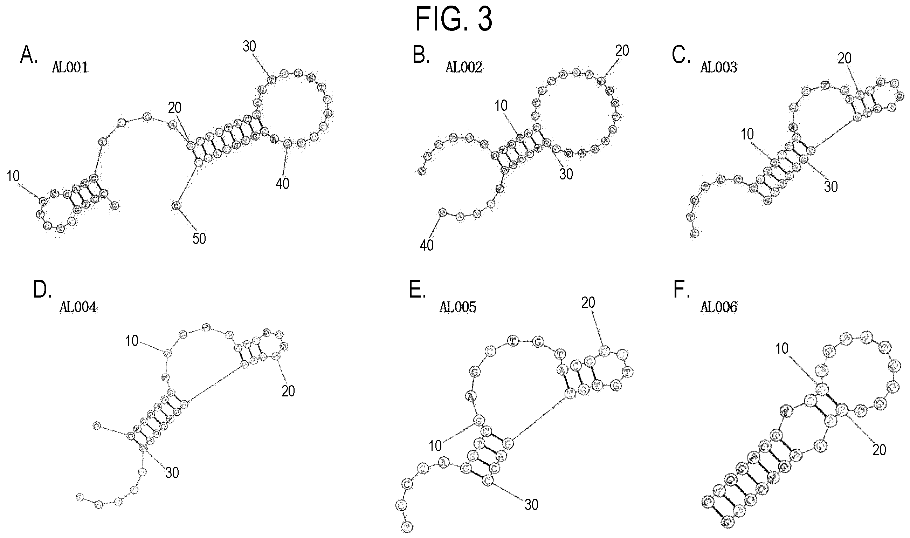

The optimization process for Clone 2 was as follows. The 2D structure of the full-length nucleic acid ligand was predicted using the Mfold web server. Based on the prediction, the length of the full-length nucleic acid ligand was reduced by truncation from both ends thereof to derive an optimized nucleic acid ligand in a minimum length. The full-length nucleic acid ligand (bold and underlined) and optimized nucleic acid ligand are listed in Table 1 as AL001, AL002, AL003, AL004, AL005, AL006, and AL007, with predicted structures of AL001, AL002, AL003, AL004, AL005, and AL006 shown in FIG. 3. The calculated and measured molecular weights for AL001 to AL007 are summarized in Table 3.

**TABLE 3**

| Nucleic acid ligand code | SEQ ID NO: | Mw Calcd. (Da) | Mw Measured (Da) |
|---|---|---|---|
| AL007 | SEQ ID NO: 1 | 24368.2466 | 24355.6250 |
| AL001 | SEQ ID NO: 2 | 17038.5797 | 17030.3340 |
| AL002 | SEQ ID NO: 3 | 13987.6530 | 13979.9082 |
| AL003 | SEQ ID NO: 4 | 12253.4802 | 12246.6211 |
| AL004 | SEQ ID NO: 5 | 12173.3623 | 12166.3096 |
| AL005 | SEQ ID NO: 6 | 10399.1654 | 10392.2246 |
| AL006 | SEQ ID NO: 7 | 10150.0077 | 10144.7578 |

### [EXAMPLE 4] Measurement of Binding Affinity of Optimized Nucleic Acid Ligand for Human Serum Albumin (Bead-based ELISA Binding Assay)

A bead-based ELISA binding assay was performed to verify the binding affinity of the optimized nucleic acid ligand for human serum albumin.

The nucleic acid ligands were synthesized using Mermade 12 or Mermade 48 (Bioautomation, USA). With Deblocking -> Coupling -> Capping -> Oxidation as one cycle, the synthesis process proceeded from the 3' end to the 5' end by sequentially adding one nucleotide.

The synthesized nucleic acid ligands were labeled at the end with biotin by mixing Biotin-11-UTP, TdT, and TdT reaction solution (Pierce, USA) and reacting at 37°C for 30 minutes. Then, treatment with EDTA inactivated the TdT enzyme.

To induce the formation of the tertiary structure of biotin-labeled nucleic acid ligands synthesized through the PCR process, the reaction was conducted while lowering the temperature gradually from 95°C to 37°C by 1°C every 10 seconds. The prepared nucleic acid ligands were allowed at 37°C for 30 minutes to bind to the human serum albumin that was 4.65-fold serially diluted starting from 200nM, and then histidine pull-down beads (Thermo Scientific, USA) were added and reacted for an additional 30 minutes at 25°C. The supernatant was removed using a magnet, the beads were washed three times with buffer SB 18, and then streptavidin-horseradish peroxidase (HRP) reagent diluted 1:10,000 was added and reacted for 30 minutes at 25°C. The supernatant was again removed using a magnet, the beads were washed three times with buffer SB 18, and then reacted with the chemiluminescent solution from the ECL Kit (Elpis, Korea). The supernatant was separated using a magnet, transferred to a white plate, and measured for fluorescence using a Glomax plate reader (Promega, USA) according to the luminescence protocol. The measurements were analyzed using SigmaPlot 11 analysis software.

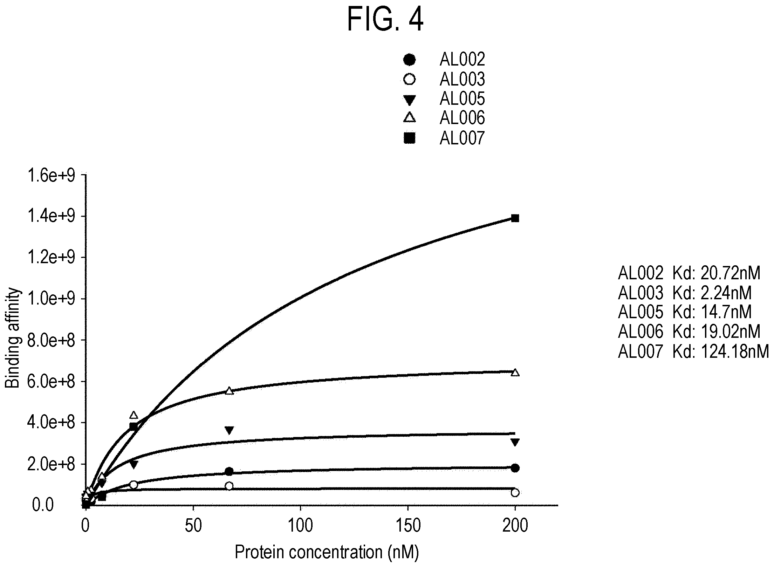

FIG. 4 shows the results of the binding affinity assay of the full-length nucleic acid ligand AL007 and optimized nucleic acid ligands AL002, AL003, AL005, and AL006 for human serum albumin. The dissociation constants (Kd values), representing the affinity between nucleic acid ligands and protein, were as follows: AL002: 20.72 nM, AL003: 2.24 nM, AL005: 14.7 nM, AL006: 19.02 nM, and AL007: 124.18 nM.

[EXAMPLE 5] Assay for in Vitro Efficacy of Human Serum Albumin-Targeting Nucleic Acid Ligand

### 5-1. Cell Culture

Miapaca-2 pancreatic cancer cell line, obtained from ATCC (USA), was used in the experiments. The cells were cultured in DMEM medium supplemented with 10% FBS and 1% antibiotics at 37°C in a 5% CO2 incubator. When the confluency reached 80% or more, passages were performed as follows. Cells washed with PBS were treated with 0.05% trypsin-EDTA for 3 minutes, and then a fresh medium was added to inactivate the trypsin. The cells were collected using a centrifuge, diluted in a new culture vessel at a 1:6 ratio, and cultured. All cells were tested for mycoplasma contamination by PCR using a kit (Biomax, Korea), and only the cells free of contamination were used for experiments.

### 5-2. Cell Growth Inhibition Effect of Nucleic Acid Ligand

The cells cultured in the aforementioned manner were seeded at a density of 1x104 cells/well into 96-well plates and cultured for 16 hours. The medium of the cells adherent to the culture plates was replaced with DMEM medium free of FBS and glutamine and cultured again for 16 hours to activate macropinocytosis. Then, the optimized nucleic acid ligand AL006, pre-bound with bovine serum albumin (BSA; Sigma, USA) for 30 minutes, was treated and cultured for 24 hours. WST reagent (Dongin LS, Korea) was added in an amount of 10 µl to each well and incubated for 2 hours. The formazan thus formed was measured using on a Glomax plate reader (Promega, Korea) according to the absorbance measurement protocol. In this experiment, bovine serum albumin was used as a negative control.

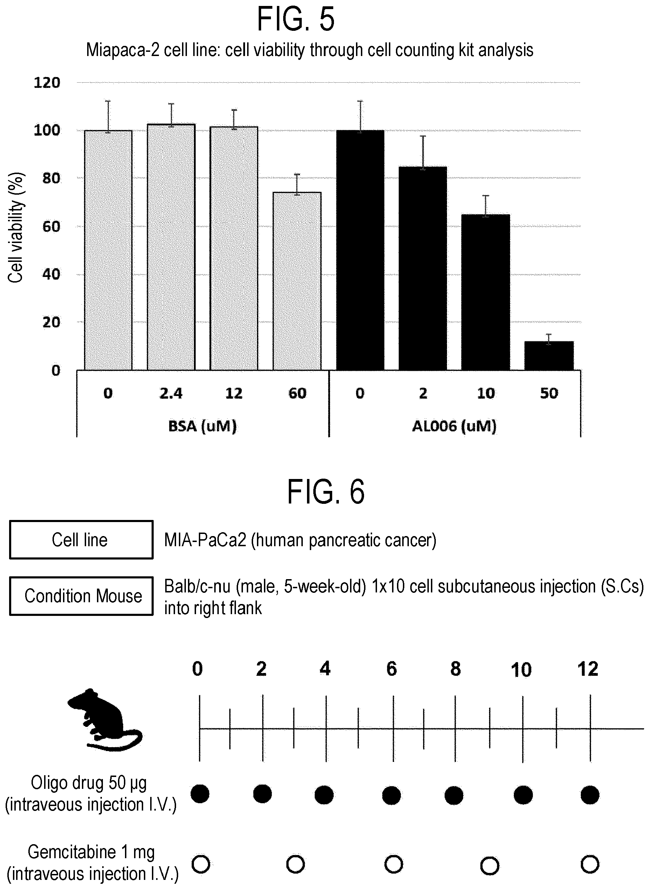

FIG. 5 shows the cell growth inhibition effect (anti-cancer efficacy) of the optimized nucleic acid ligand AL006 on the Miapaca-2 pancreatic cancer cell line. According to FIG. 5, the optimized nucleic acid ligand AL006 showed cell growth inhibition effects significantly decreased in a dose-dependent manner, while the bovine serum albumin negative control group, which was not treated with the nucleic acid ligand, showed insignificant inhibition effects.

### [EXAMPLE 6] Assay for in Vivo Anticancer Efficacy of Pharmaceutical Composition Containing Nucleic Acid Ligand in Pancreatic Cancer Xenograft Animal Model

### 6-1. Animal Model Preparation

Male Balb/c-nu mice (5 weeks old) were purchased from ORIENT BIO (Seongnam, Korea). All animal experiments were conducted in accordance with the regulations of the Institutional Animal Care and Use Committee (IACUC) of Seoul National University.

Miapaca-2 cell line (1X106 cells) cultured using the method described in section 5-1 was mixed with 100µl of Matrigel mixture (1XPBS: Matrigel = 50:50 vol.) and subcutaneously injected into the right flank of the prepared Balb/c-nu male mice. When the tumor was grown to the size of about 30mm3 after subcutaneous injection, the efficacy in tumor suppression was compared between intravenous injections of the positive control drug (gemcitabine) and the optimized nucleic acid ligand AL006.

### 6-2. Tumor Suppression Efficacy of Human Serum Albumin-Targeting Nucleic Acid Ligand in Pancreatic Cancer Xenograft Animal Model

Each experimental group consisted of 5 animals. Gemcitabine (1mg) was administered once every 3 days, 5 times in total (Day 0, 3, 6, 9, and 12), and the optimized nucleic acid ligand AL006 (50µg) was administered once every 2 days, 7 times in total (Day 0, 2, 4, 6, 8, 10, and 12). Tumor size was measured every 2 days using the formula [1/2 x longest diameter x shortest diameter] (see FIG. 6).

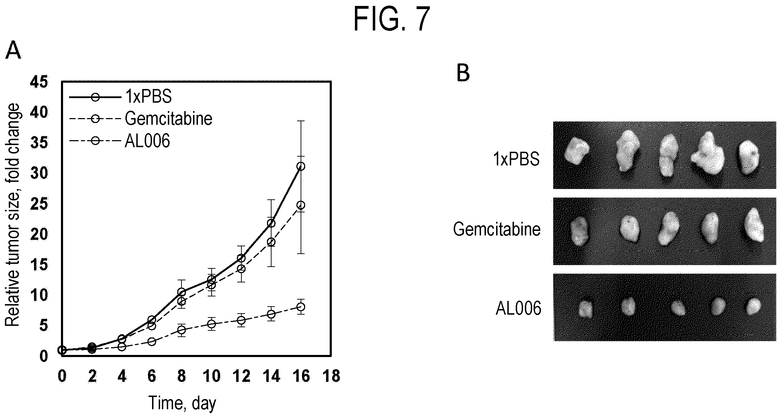

The results of tumor suppression efficacy (anti-cancer efficacy) are shown in Figure 7. Compared to the positive control drug gemcitabine, the optimized nucleic acid ligand AL006 exhibited significantly superior tumor suppression effects.

### 6-3. In Vivo Stability of Human Serum Albumin-Targeting Nucleic Acid Ligand in Pancreatic Cancer Xenograft Animal Model

After the termination of drug administration, the mice were sacrificed and organs were excised therefrom. The organs were weighed and the blood was analyzed for the toxicity attributed to drug administration.

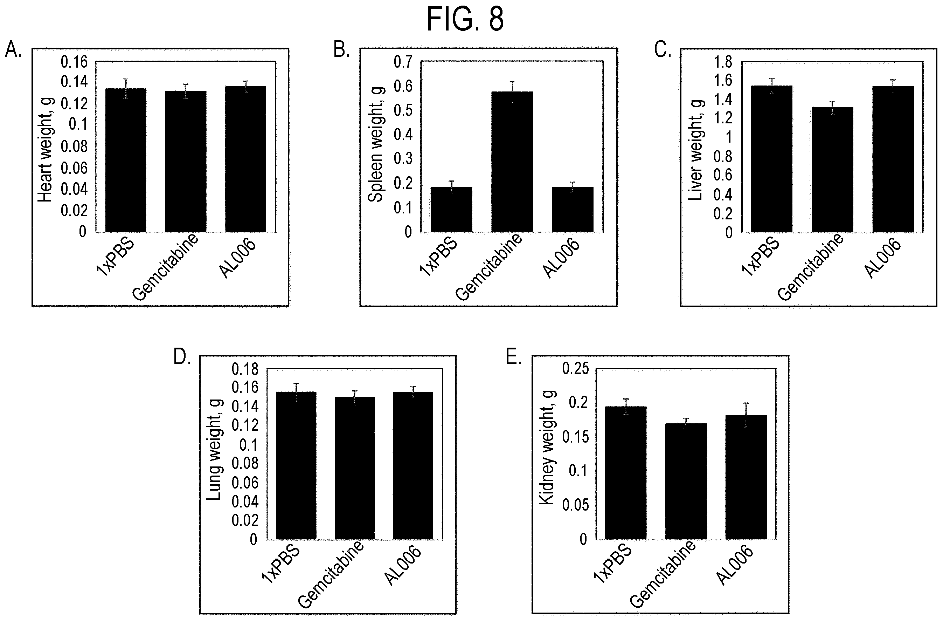

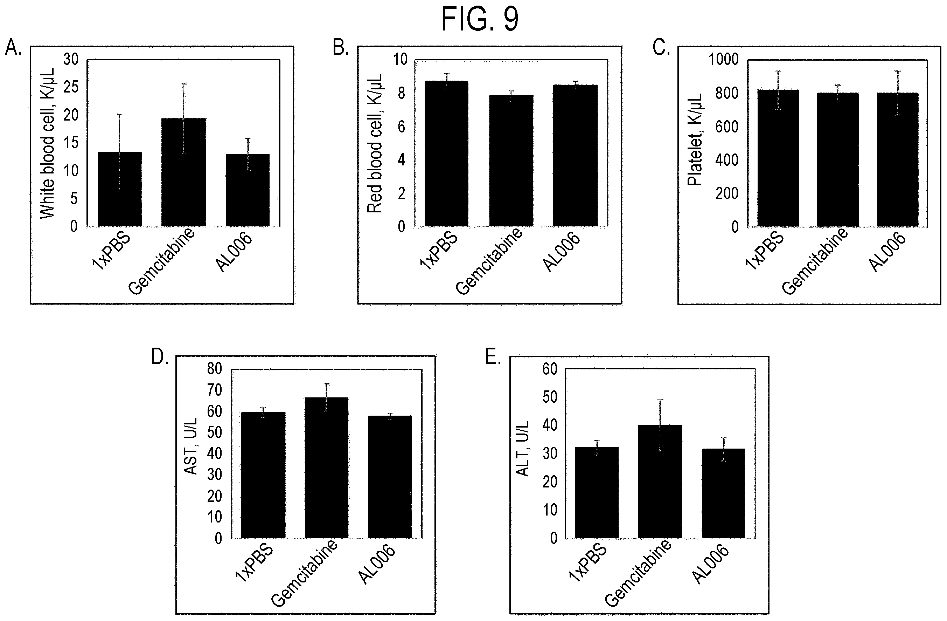

The assay results for in vivo stability are shown in FIGS. 8 and 9. The gemcitabine-treated group, which was the positive control drug group, showed side effects such as splenomegaly and decreased liver weight, while the optimized nucleic acid ligand AL006-treated group showed no side effects in the liver, spleen, heart, lungs, kidneys, etc.

Specifically, the increase in liver damage indicators AST and ALT were observed in the gemcitabine-treated group, and side effects such as decreased white blood cell and platelet counts were noted. In contrast, the optimized nucleic acid ligand AL006-treated group did not show abnormal values in liver damage indicators, white blood cells, platelets, and red blood cells.

### [EXAMPLE 7] Assay for Efficacy of Human Serum Albumin-Targeting Nucleic Acid Ligand in Triple-Negative Breast Cancer Syngeneic Animal Model

The selection of in-vivo experimental models is crucial for evaluating the anticancer efficacy against breast cancer, particularly triple-negative breast cancer (TNBC). Due to the complex immunological and physiological actions in the human body, the use of a model that closely resembles the human environment is necessary to reliably infer anticancer efficacy in humans from animal preclinical experimental results. The model selection is especially important for TNBC, which is invasive, metastatic, and recurrent, with a high probability of early recurrence and mortality in remote organs.

Currently available preclinical spontaneously metastatic TNBC orthotopic murine models include the immunodeficient human-derived MDA-MB-231 model and the immunocompetent murine 4T1 model. The human-derived MDA-MB-231 cell line, which is a xenograft in mice, requires the use of NOD/SCID or nude mice. The xenografted cancer cells are not inherently invasive or aggressive unlike those in mice, as the host is not human. Therefore, Matrigel is typically used during xenografting because human-derived cancer cells do not readily engraft in mice. The MDA-MB-231 model does not grow as quickly in mice as mouse cells like 4T1, CT26, MC38, B16F10, etc. The relative lack of invasiveness/aggressiveness in the mouse model makes metastasis occur less frequently therein. NOD/SCID or nude mice that need to be used for xenografting do not give reliability for assay results of anticancer efficacy in humans because their immune system is deleted or do not operate normally due to the lack of some or all immune cells. This is particularly true for invasive and metastatic cancers like TNBC, as the model might not fully replicate and evaluate the cancer's occurrence and anticancer mechanisms. Additionally, since immune cells contribute to the mechanism of cancer metastasis, the MDA-MB-231 model, which necessitates the use of immunodeficient mice, has limitations in evaluating efficacy against TNBC compared to the 4T1 model and may not yield accurate evaluation results. Therefore, in this experiment, the 4T1 mouse model was used to evaluate the prophylactic or therapeutic effect of the nucleic acid ligand of the present disclosure on primary and metastatic TNBC (i.e., cancer metastasis to other organs or metastatic cancer occurring in other organs even after surgical removal). In other words, the 4T1 mouse model is not only a primary TNBC model for targeted treatment of the primary tumor that grows after injecting 4T1 tumor cells into the mammary fat pad (4^{th} fat pad) of mice, but also a metastatic breast cancer model, in which the 4T1 tumor cells grown as above are used. When the primary tumor is completely removed surgically, the remaining tumor cells spontaneously metastasize to other organs, especially the lungs, creating a breast cancer spontaneous metastasis model. In this regard, when 4T1 cells tagged with the luciferase reporter gene are employed, imaging can be performed at various time points using luciferin and an in vivo imaging system (IVIS). Thus, 4T1 mouse model is a useful model for observing the effects of anticancer drugs on primary tumor treatment and breast cancer metastasis treatment.

### 7-1 Cell Culture

The 4T-1 Lu2 triple-negative breast cancer cell line was obtained from ATCC (USA) and cultured using ATCC modified RPMI-1640 (Thermo Scientific, USA) supplemented with 10% FBS and 1% antibiotics. The passages were conducted in the same manner as in section 5-1.

### 7-2 Animal Model Preparation

Female Balb/c mice (7 weeks old) were purchased from ORIENT BIO (Seongnam, Korea), and all animal experiments were conducted in accordance with the regulations of the Institutional Animal Care and Use Committee (IACUC) of Sungkyunkwan University.

The 4T-1 Lu2 cells (5X105 cells), cultured as described in section 7-1, were diluted in 30µl of 1X PBS (Hyclone) and injected into the mammary fat pad (4th fat pad) of the prepared female Balb/c mice. When the injected cells proliferated to form a tumor of approximately 60mm3, the mice were divided into groups with and without tumor resection. The groups were then treated with positive control drug (gemcitabine), negative control drug (HER2-targeting nucleic acid ligand; SEQ ID NO: 23), and optimized nucleic acid ligand AL006 via intraperitoneal injection (IP) to compare the tumor suppression efficacy.

### 7-3 Suppressive Efficacy against Metastasis and Recurrence

### 7-3-1 Metastasis and recurrence suppression efficacy in non-resected tumor animal model

Each experimental group consisted of 5 animals. The positive control drug, gemcitabine (1mg), and the test drugs, optimized nucleic acid ligand AL006 (30µg, 100µg, 300µg), were injected every two days for a total of 6 times (Day 0, 2, 4, 6, and 8). Tumor size was measured every two days using the formula [longest tumor axis x (shortest tumor axis)2 x 0.5].

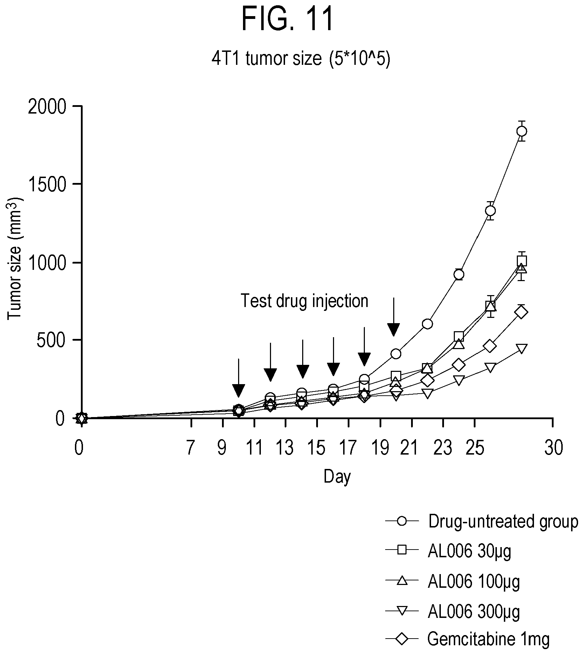

The results of the tumor suppression efficacy in the non-resected tumor experiment are depicted in FIG. 11. As can be seen, the optimized nucleic acid ligand AL006 demonstrated tumor suppression effects in a dose-dependent manner with superiority to the positive control drug gemcitabine. Notably, 300 µg of the optimized nucleic acid ligand AL006 demonstrated far higher tumor suppression efficacy than the positive control drug, gemcitabine.

### 7-3-2 Suppressive efficacy against metastasis and recurrence in resected tumor animal model

For the resected tumor animal model, when the injected cells proliferated to form a tumor of approximately 60mm3, the tumor was surgically removed. The positive control drug, gemcitabine (1mg), the negative control drug, HER2-targeting nucleic acid ligand (300µg), and the test drugs, optimized nucleic acid ligand AL006 (30µg, 300µg, 600µg), were administered. For the low-dose treatment groups (30µg), intraperitoneal injections were given every two days for a total of 6 times (Day 0, 2, 4, 6, 8). For the high-dose treatment groups (300µg, 600µg), injections were given every two days for a total of 15 times (Day 0, 2, 4, 6, 8, 10, 12, 14, 16, 18, 20, 22, 24, 26, and 28) via intraperitoneal injection.

To confirm the efficacy of metastasis and recurrence suppression, the animals were imaged using an in-vivo imaging analyzer for experimental animals (IVIS Spectrum) at approximately 7-day intervals until 50 days post-treatment. Each animal was injected with luciferin at a concentration of 150mg/kg, and imaging with IVIS was performed 10 minutes post-injection.

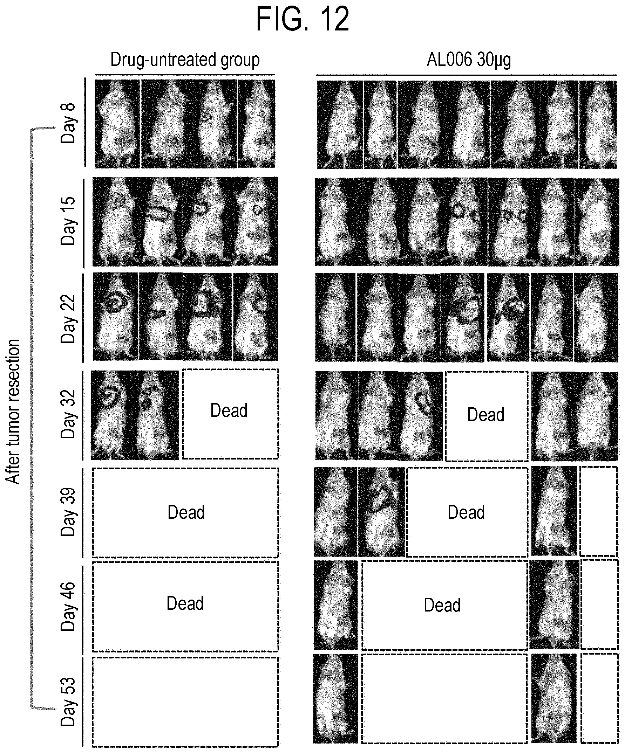

The results of metastasis and recurrence suppression in the low-dose treatment group are shown in FIG. 12. While all animals in the untreated group died by Day 39 post-tumor resection, some individuals in the group treated with 30µg of the optimized nucleic acid ligand AL006 survived until Day 53.

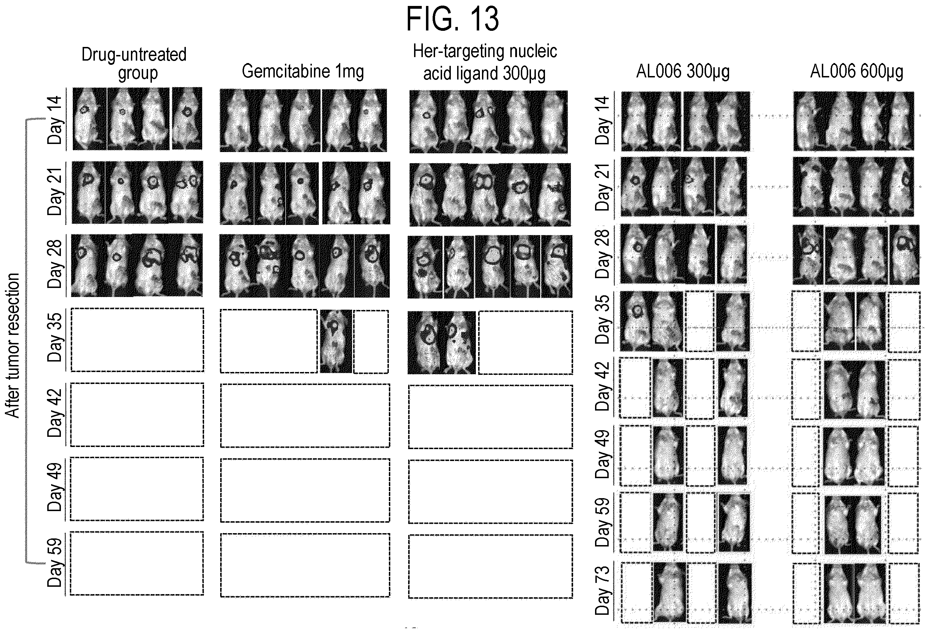

To compare the effects of optimized nucleic acid ligand AL006 on tumor metastasis and recurrence, additional groups receiving high doses (300µg, 600µg), along with the positive control group (gemcitabine 1mg) and negative control group (HER2-targeting nucleic acid ligand 300µg), were included. The results are shown in FIG. 13. All animals in the positive (gemcitabine 1mg) and negative (HER2-targeting nucleic acid ligand 300µg) control groups as well as in the untreated group died by Day 42 post-tumor resection. In the high-dose treatment groups of the optimized nucleic acid ligand AL006, some individuals survived until Day 73, and no metastasis or recurrence was observed in the IVIS results.

## Claims

1. A non-natural nucleic acid ligand, which binds to human serum albumin (HSA) and comprises the nucleotide sequence of SEQ ID NO: 8 (CAGG6CGAGC6G6ACGCG6G6G6GACC) [wherein the portions marked as 6 are each 5-[N-(1-naphthylmethyl)carboxamide]-2'-deoxyuridine (Nap-dU)].

2. The non-natural nucleic acid ligand of claim 1, wherein the non-natural nucleic acid ligand includes a nucleic acid sequence selected from SEQ ID NOS: 1 to 8.

3. A pharmaceutical composition for prevention or treatment of cancer, the composition comprising a non-natural nucleic acid ligand or a pharmaceutically acceptable salt thereof that binds to human serum albumin (HSA),

4. The pharmaceutical composition of claim 3, wherein the non-natural nucleic acid ligand comprises the nucleic acid sequence of SEQ ID NO: 8 (CAGG6CGAGC6G6ACGCG6G6G6GACC) [wherein the portions marked as 6 are each 5-[N-(1-naphthylmethyl)carboxamide]-2'-deoxyuridine (Nap-dU)] and the composition comprises at least one of the non-natural nucleic acid ligand.

5. The pharmaceutical composition of claim 4, wherein the non-natural nucleic acid ligand comprising the nucleic acid sequence of SEQ ID NO: 8 has a nucleic acid sequence selected from SEQ ID NOS: 1 to 8.

6. The pharmaceutical composition of claim 3, wherein the cancer is a primary or metastatic cancer.

7. The pharmaceutical composition of claim 3, wherein the pharmaceutical composition is administered intravenously, intramuscularly, intraarterially, intraperitoneally, intranasally, intravaginally, intravesically, intradermally, transdermally, topically, or subcutaneously to a subject in need thereof.

8. The pharmaceutical composition of claim 3, wherein the pharmaceutical composition is administered after surgical resection of a cancer tissue.

9. The pharmaceutical composition of claim 3, wherein the cancer is breast cancer, pancreatic cancer, lung cancer, colon cancer, ovarian cancer, endometrial cancer, cervical cancer, bladder cancer, small intestine cancer, testicular cancer, thyroid cancer, liver cancer, urothelial carcinoma, gallbladder and biliary tract cancer, skin cancer, gastric cancer, brain tumor, kidney cancer, and/or acute myeloid leukemia.
